# EUROPEAN PATENT SPECIFICATION

(11) **EP 0 648 827 B1**
(45) Date of publication and mention of the grant of the patent: **22.12.1999**
(21) Application number: 94115525.1
(22) Date of filing: 01.10.1994
(51) Int. Cl.: C09K 19/12, C09K 19/20, C09K 19/38, C09K 19/32, C09K 19/34

(54) **Reactive liquid crystal compounds**
Reaktive Flüssigkristallverbindungen
Composés cristallins liquides réactifs

(30) Priority: 15.10.1993 EP 93116679
(43) Date of publication of application: 19.04.1995
(73) Proprietor: MERCK PATENT GmbH, 64293 Darmstadt (DE)
(72) Inventor: Parri, Owain Llyr, Dr., Poole, Dorset BH15 2LN (GB); Coates, David, Dr., Wimborne, Dorset BH12 3SW (GB); Greenfield, Simon, Dr., Poole, Dorset BH17 7YA (GB); Bonny, Ian, Poole, Dorset BH17 7UZ (GB)

(56) References cited:
- EP-A- 0 293 870
- EP-A- 0 484 972
- EP-A- 0 501 563
- WO-A-93/22397
- WO-A-94/08268
- ADVANCED MATERIALS, vol.5, no.2, February 1993 pages 107 - 109 K.GEIBEL ET AL. 'in situ photopolymerized, oriented liquid-crystalline diacrylates with high thermal conductivities'

## Description

The invention relates to
reactive liquid crystal compounds of formula I

R¹-P-X-(A¹-Z¹)ₙ-A²-R² I

wherein
- R¹: is CH₂ = CW¹-COO-, CH₂ = CH-, 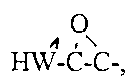 HW¹N-, HS-CH₂-(CH₂)m-COO- with W¹ being H, Cl or alkyl with 1-5 C atoms and m being 1-7,
- P: is alkylene with up to 12 C atoms, it being also possible for one or more CH₂ groups to be replaced by O,
- X is -O-, -S-, -COO-, -OCO- or a single bond,
- R²: is alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not directly linked to one another, or alternatively R² has one of the meaning given for R¹-P-X or is CN, F, Cl or -V-Q-T wherein V is -O-, -S- or a single bond, Q is -CH₂-, -CHF-, -CF₂-, and T is F or Cl,
- n: is 1 or 2,
- A¹, A²: are independently from each other 1,4-phenylene which is unsubstituted or mono- or polysubstituted by F, Cl, CN or -W²-CᵣHₛF₂ᵣ₊₁₋ₛ, and one of A¹ and A² may also denote 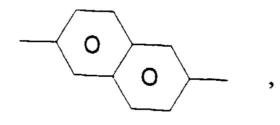 pyridine-2,5-diyl or pyrimidine-2,5-diyl,
- Z¹: is independently from each other -CH₂-CH₂- or a single bond, in case n=2 both the moieties A¹ as well as both the moieties Z¹ are having the meaning given above, independently from each other,
- r: is 1 or 2,
- s: is 0, 1, 2, 3, 4 or 5,
- W²: is a single bond, -O-, -S- or -CO-,
with the provisos
(i) that at least one of A¹ and A² is 1,4-phenylene which is mono- or polysubstituted by Cl, CN or -W²-CᵣHₛF₂ᵣ₊₁₋ₛ, it also being possible for this substituted 1,4-phenylene group to be additionally substituted by one or more F-atoms, and
(ii) that in case n=2 at least one of A¹ and A² is double substituted or at least two of A¹ and A² are substituted,
characterized in that said compound is laterally di- or higher substituted by CN or -W²-CᵣHₛF₂ᵣ₊₁₋ₛ ,
reactive liquid crystal compounds of formula I2

R¹-P-X-A¹-Z¹-A²-R² I2

wherein
- R¹: is CH₂=CW¹-COO-, CH₂=CH-, 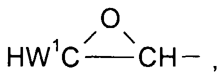 HW¹N- or HS-CH₂-(CH₂)ₘ-COO-, with W being H, Cl or alkyl with 1-5 C-atoms and m being 1-7,
- P: is alkylene with up to 12 C atoms, it being also possible for one or more CH₂ groups to be replaced by O in such a manner that oxygen atoms are not directly linked to one another,
- X: is -O-, -S-, -COO-, -OCO- or a single bond,
- R²: is an alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not linked directly to one another, or alternatively R² has one of the meanings given for R¹-P-X or is CN, F, Cl or -V-Q-T wherein V is-O-, -S- or a single bond, Q is -CH₂-, -CHF-, -CF₂-, and T is F or Cl,
- A¹, A²: are independently from each other 1,4-phenylene which is unsubstituted or mono- or polysubstituted by F, Cl, CN or -W²-CᵣHₛF₂ᵣ₊₁₋ₛ, and one of A¹ and A² may also denote pyridine-2,5-diyl or pyrimidine-2,5-diyl,
- Z¹: is independently from each other -CH₂-CH₂- or a single bond,
- r: is 1 or 2,
- s: is 0, 1, 2, 3, 4 or 5,
- W²: is a single bond, -O-, -S- or -CO-,
with the proviso that at least one of A¹ and A² is 1,4-phenylene which is monosubstituted by Cl, CN or -W²-CᵣHₛF₂ᵣ₊₁₋ₛ, it also being possible for this substituted 1,4-phenylene group to be additionally substituted by one or more F-atoms,
characterized in that said compound is laterally monosubstituted by -Cl, -CN or -W²-CᵣHₛF₂ᵣ₊₁₋ₛ,
reactive liquid crystal compounds of formula I3

R¹-P-X-A¹-Z¹-A¹-Z¹-A²-R² I3

wherein
- R¹: is CH₂=CW¹-COO-, CH₂=CH-, 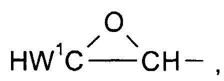 HW¹N- or HS-CH₂-(CH₂)ₘ-COO-, with W¹ being H, Cl or alkyl with 1-5 C-atoms and m being 1-7,
- P: is alkylene with up to 12 C atoms, it being also possible for one or more CH₂ groups to be replaced by O in such a manner that oxygen atoms are not directly linked to one another,
- X: is -O-, -S-, -COO-, -OCO- or a single bond,
- R²: is an alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not linked directly to one another, or alternatively R² has one of the meanings given for R¹-P-X or is CN, F, Cl or -V-Q-T wherein V is-O-, -S- or a single bond, Q is -CH₂-, -CHF-, -CF₂-, and T is F or Cl,
- A¹, A²: are independently from each other 1,4-phenylene which is unsubstituted or mono- or polysubstituted by F, Cl, CN or -W²-CᵣHₛF₂ᵣ₊₁₋ₛ, and one of A¹ and A² may also denote 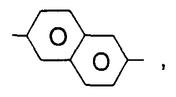 pyridine-2,5-diyl or pyrimidine-2,5-diyl,
- Z¹: is independently from each other -CH₂-CH₂- or a single bond, in case n=2 both the moieties A¹ as well as both the moieties Z¹ are having the meaning given above, independently from each other,
- r: is 1 or 2,
- s: is 0, 1, 2, 3, 4 or 5,
- W²: is a single bond, -O-, -S- or -CO-,
with the proviso that at least one of A¹ and A² is 1,4-phenylene which is mono- or polysubstituted by Cl, CN or -W²-CᵣHₛF₂ᵣ₊₁₋ₛ, it also being possible for this substituted 1,4-phenylene group to be additionally substituted by one or more F-atoms,
characterized in that said compound is laterally monosubstituted by -Cl, -CN or -W²-CᵣHₛF₂ᵣ₊₁₋ₛ.,
with the proviso that compounds being only monosubstituted by Cl are excluded.

The invention furthermore relates to the preparation of such compounds and to their use in electrooptical scattering systems and for the preparation of oriented liquid crystal polymers.

Reactive liquid crystal compounds can be polymerized in situ, whilst in their liquid crystal phase, to give highly crosslinked anisotropic films which can be used, for example, as polarizing beam splitters (see, for example, EP 0,428,213). Reactive liquid crystal compounds have furthermore been proposed for electrooptical scattering systems (see, for example, EP 0,451,905).

Reactive liquid crystal diesters of formula 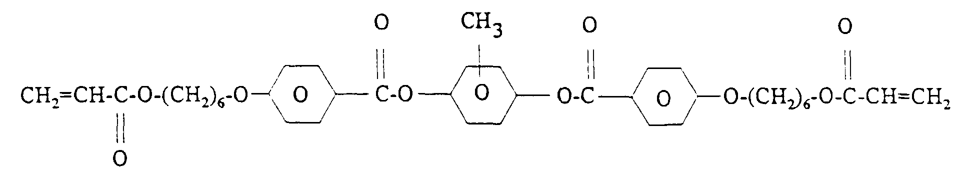 which are laterally substituted by a methyl group are mentioned in EP 0,331,233. These reactive liquid crystalline compounds often exhibit, however, rather high melting points and disadvantageous values of the birefringence.

Furthermore, reactive liquid crystals, polymers obtained thereof and their uses are disclosed in the prior art documents EP 0 501 563 A1, EP 0 484 972 A2, WO 93/22397 Al, WO 94/08268 A1 and by K. Geibel et al., Advanced Materials 5, 2 (1993), p. 107-109.

In view of the broad range of applications of reactive liquid crystal compounds it was desirable to have available further compounds of this type which fulfill the various requirements such as a reasonably low melting point, a high birefringence, a broad mesogenic range and preferably an enantiotropic nematic range to a high degree.

It was an object of the present invention to provide new reactive liquid crystalline compounds with advantageous properties thus extending the pool of reactive liquid crystal compounds available to the expert. Other objects of the present invention can be taken from the following detailed specification.

The present invention thus relates to reactive liquid crystal compounds of formula I and to their use in electrooptical systems of scattering type and for the preparation of oriented liquid crystal polymers. The invention furthermore relates to the preparation of compounds according to formula I.

Above and below, the term reactive liquid crystalline compounds refers to reactive rod-like molecules of formula I or other rod-like reactive compounds which may be enantiotropic, monotropic or isotropic, preferably, however, enentiotropic or monotropic.

In the compounds of formula I, A¹ and A² can be independently from each other an unsubstituted or a substituted 1,4-phenylene group of formula 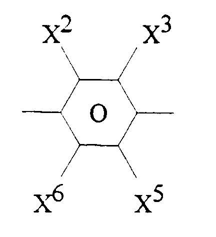 X², X³, X⁵ and X⁶ can be independently from each other H, F, Cl, CN or -W²-CᵣHₛF₂ᵣ₊₁₋ₛ.

In the following, for the sake of simplicity, the following notation will be used:
Phe. 2 X² 3 X³ 5 X⁵ 6 X⁶ is a 1,4-phenylene group carrying in 2-position the group X², in 3-position the group X³ etc.; in case X², X³, X⁵ and/or X⁶, denote H, this will not be specified in above notation, i.e. only true substitutions will be listed. Thus Phe, for example, is an unsubstituted 1,4-phenylene group while Phe.2F 5 Cl is a 2-fluoro-5-chloro-1,4-phenylene group. Furthermore, Pyr is pyrimidine-2,5-diyl, Pyd is pyridine -2,5-diyl and Nap is a naphthalene-2,6-diyl group. The notation Pyr and Pyd in each case include the 2 possible positional isomers.

The compounds according to formula I comprise 2- and 3-ring compounds (n = 1 or 2) of formula I2 and I3:

R¹-P-X-A¹-Z¹-A²-R² I2

R¹-P-X-A¹-Z¹-A¹-Z¹-A²-R² I3

In the 3-ring compounds of formula 13, the ring groups A¹ can be chosen independently from each other.

Compounds according to formula I which are laterally monofluorinated often exhibit smectic phases ... and are not covered by the present invention. Contrary to this, compounds according to formula I which are laterally monosubstituted by -Cl, -CN or -W²-CᵣH₂ᵣ₊₁₋ₛ and, in particular, by -Cl,-CH₃, -O-CH₃, -CF₃, -OCF₃, -CH₂F, -OCH₂F, -CHF₂, -OCHF₂, -C₂H₅, -S-CH₃, -S-CF₃ are characterized by advantageous properties and the tendency to form smectic phases is considerably reduced.

Compounds according to formula I which are laterally di- or higher substituted by -F, -Cl, -CN and/or -W²-CᵣH₂ᵣ₊₁₋ₛ and, in particular, by -F, -Cl, -CN, -CH₃, -OCH₃, -CF₃ and/or -OCF₃ are furthermore preferred. This substitution pattern distinctly supresses smectic phases and promotes formation of the nematic phase.

3-ring compounds according to formula I wherein both bridging groups Z¹ are -COO- and/or -OCO- are often characterized by an insufficient stability and these compounds are not covered by the present invention.

Especially preferred is a smaller group of 2-ring compounds exhibiting the following structures for -A¹-Z¹-A²-;

-Phe.2CH₃-Phe- I2-1

-Phe.3CH₃-Phe- I2-2

-Phe.2Cl-Phe- I2-3

-Phe.3Cl-Phe- I2-4

-Phe.2CN-Phe- I2-5

-Phe.3CN-Phe- I2-6

-Phe.2Cl3F-Phe- I2-8

-Phe.2OCH3-Phe- I2-9

-Phe.3OCH3-Phe- I2-10

-Phe.2C₂H₅-Phe- I2-11

-Phe.3C₂H₅-Phe- I2-12

-Phe.2CF₃-Phe- I2-13

-Phe.3CF₃-Phe- I2-14

-Phe.2CH₃-Phe.2CH₃- I2-15

-Phe.2CH₃-Phe.3CH₃- I2-16

-Phe.2CH₃-Phe.2F- I2-17

-Phe.2CH₃-Phe.3F- I2-18

-Phe.2CH₃-Nap- I2-19

-Phe.2Cl-Nap- I2-20

-Phe.3CH₃-Nap- I2-21

-Phe.3Cl-Nap- I2-22

-Phe.2CH₃-Pyr- I2-23

-Phe.2Cl-Pyr- I2-24

-Phe.2CH₃-Pyd- I2-25

-Phe.2Cl-Pyd- I2-26

-Phe.2CH₃-CH₂CH₂-Phe- I2-27

-Phe.3CH₃-CH₂CH₂-Phe- I2-28

-Phe.2Cl-CH₂CH₂-Phe- I2-29

-Phe.3Cl-CH₂CH₂-Phe- I2-30

-Phe.2CN-CH₂CH₂-Phe- I2-31

-Phe.3CN-CH₂CH₂-Phe- I2-32

-Phe.2Cl3F-CH₂CH₂-Phe- I2-34

-Phe.2OCH₃-CH₂CH₂-Phe- I2-35

-Phe.2OCF₃-CH₂CH₂-Phe- I2-36

-Phe.3OCH₃-CH₂CH₂-Phe- I2-37

-Phe.2C₂H₅-CH₂CH₂-Phe- I2-38

-Phe.2C₂H₅-CH₂CH₂-Phe- I2-39

-Phe.2CF₃-CH₂CH₂-Phe- I2-40

-Phe.3CF₃-CH₂CH₂-Phe- I2-31

The 3-ring compounds according to formula 13 preferably exhibit the following structures for -A¹-Z¹-A¹-Z¹-A²:

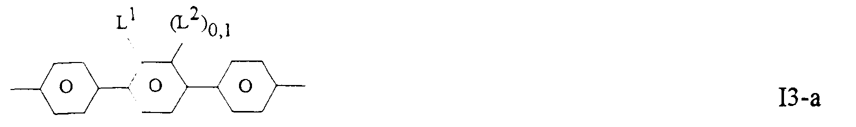

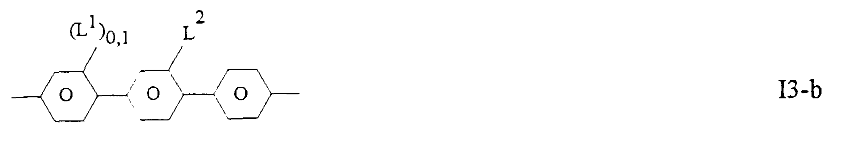

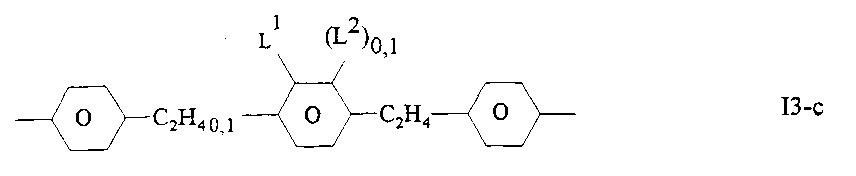

In these structures I3-a, I3-b and I3-c, L¹ and L² denote independently from each other -Cl, -F, -CN and W²-CᵣH₂ᵣ₊₁₋ₛ and, in particular, -Cl, -F, -CN, -CH₃, -OCH₃, -CH₂F, -OCH₂F, -CHF₂, -OCHF₂, -CF₃, -OCF₃ and/or -C₂H₅.

Especially preferred are the following patterns:

-Phe-Phe.2CH₃-Phe- I3-a-1

-Phe-Phe.2CN-Phe- I3-a-3

-Phe-Phe.2CF₃-Phe- I3-a-4

-Phe-Phe.2OCF₃-Phe- I3-a-5

-Phe-Phe.2OCH₃-Phe- I3-a-6

-Phe-Phe.2C₂H₅-Phe- I3-a-7

-Phe-Phe.2CH₃3F-Phe- I3-a-8

-Phe-Phe.2F3CF₃-Phe- I3-a-11

-Phe-Phe.3CH₃-Phe- I3-b-1

-Phe-Phe.3CN-Phe- I3-b-3

-Phe-Phe.3CF₃-Phe- I3-b-4

-Phe-Phe.3OCF₃-Phe- I3-b-5

-Phe-Phe.3OCH₃-Phe- I3-b-6

-Phe-Phe.3C₂H₅-Phe- I3-b-7

-Phe.3F-Phe.3CH₃-Phe- I3-b-9

-Phe.3Cl-Phe.3CH₃-Phe- I3-b-11

-Phe-Phe.2CH₃-Phe.3Cl- I3-b-16

-Phe-Phe.3CH₃-Phe.3Cl- I3-b-17

-Phe-Phe.2CH₃-Phe.2Cl- I3-b-18

-Phe-Phe.3CH₃-Phe.2Cl- I3-b-19

-Phe-Phe.2Cl-Phe.3CN- I3-b-24

-Phe-Phe.3Cl-Phe.3CN- I3-b-25

-Phe-Phe.2Cl-Phe.2CN- I3-b-26

-Phe-Phe.3Cl-Phe.2CN- I3-b-27

-Phe-Phe.2CH₃-Phe.3CN- I3-b-28

-Phe-Phe.3CH₃-Phe.3CN- I3-b-29

-Phe-Phe.2CH₃-Phe.2CN- I3-b-30

-Phe-Phe.3CH₃-Phe.2CN- I3-b-31

-Phe-Phe.3F-Phe.3CN- I3-b-32

-Phe-Phe.2F-Phe.3 CN- I3-b-33

-Phe-Phe.3F-Phe.2CN- I3-b-34

-Phe-Phe.2F-Phe.2CN- I3-b-35

-Phe-Phe.2CH₃-C₂H₄-Phe- I3-c-1

-Phe-Phe.2Cl-C₂H₄-Phe- I3-c-2

-Phe-Phe.2CN-C₂H₄-Phe- I3-c-3

-Phe-Phe.2CHF₂-C₂H₄-Phe- I3-c-4

-Phe-Phe.2OCH₃-C₂H₄-Phe- I3-c-5

-Phe-Phe.2CF₃-C₂H₄-Phe- I3-c-6

-Phe-Phe.2OCF₃-C₂H₄-Phe- I3-c-7

-Phe-Phe.2C₂H₅-C₂H₄-Phe- I3-c-8

-Phe-Phe.2CH₃3F-C₂H₄-Phe- I3-c-9

-Phe-Phe.2F3CF₃-C₂H₄-Phe- I3-c-12

-Phe-C₂H₄-Phe.2CH₃-C₂H₄-Phe- I3-c-13

-Phe-C₂H₄-Phe.2CN-C₂H₄-Phe- I3-c-15

-Phe-C₂H₄-Phe.2OCH₃-C₂H₄-Phe- I3-c-16

-Phe-C₂H₄-Phe.2CF₃-C₂H₄-Phe- I3-c-17

-Phe-C₂H₄-Phe.2OCF₃-C₂H₄-Phe- I3-c-18

It was observed that the stability of 3-ring compounds wherein one of the 2 groups Z¹ is -COO- or -OCO- while the other denotes a single bond, can be increased if the compound is laterally di- or higher substituted, particularly di-substituted by -Cl, -F, -CN and/or -CH₃.

In the compounds of formula I R¹ is CH₂ = CW¹-COO-, CH₂ = CH-, 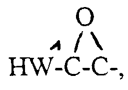 HW¹N-, HS-CH₂-(CH₂)ₘ-COO- with W¹ being H, Cl or alkyl with 1-5 C atoms and m being 1-7.

Preferably, R¹ is a vinyl group, an acrylate group, an epoxy group, an amino group or a mercapto group, and especially preferred are the following means of R¹:

CH₂=CH-COO- R¹-1

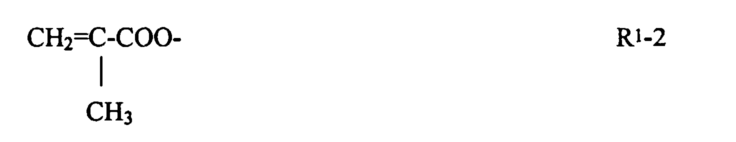

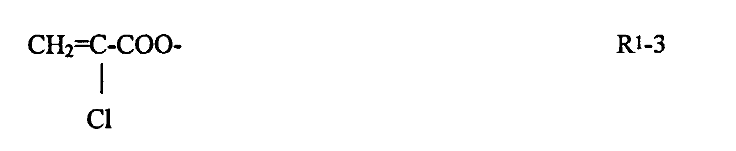

CH₂=CH- R¹-4

H₂N- R¹-5

H(alkyl)N- R¹-6

HS-CH₂-(CH₂)ₘ-COO- R¹-7

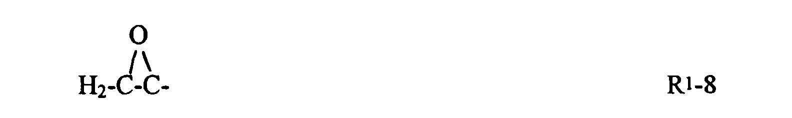

with alkyl denoting C₁-C₃-alkyl and m being 1-5.

In the compounds of formula I, the spacer-type group P is alkylene with up to 12 C atoms, it also being possible for one or more non adjacent CH₂ groups to be replaced by O.

In case P is alkylene, P may be straight-chain or branched. P especially preferred is ethylene, propylene, butylene, 1-methyl-propylene, 2-methyl-propylene, pentylene, 1-methyl-butylene, 2-methyl-butylene, hexylene, 2-ethyl-butylene, 1,3-dimethyl-butylene, heptylene, 1-methythexylene, 2-methylhexylene, 3-methylhexylene, 4-methylhexylene, 5-methylhexylene, 6-methylhexylene, octylene, 3-ethyl-hexylene, nonylene, 1-methyloctylene, 2-methyloctylene, 7-methyloctylene, decylene, undecylene, 2-methylundecylene, 2,7,5-trimethyl-nonylene or 3-propyl-nonylene.

In case P is mono- or polyoxaalkylene, P may be straightchain or branched. In particular, P is 1-oxa-ethylene, 1-oxa-propylene, 2-oxapropylene, 1-oxabutylene, 2-oxabutylene, 1,3-dioxabutylene, 1-oxa-pentylene, 2-oxapentylene, 3-oxy-pentylene, 2-oxa-3-methyl-butylene, 1-oxahexylene, 2-oxa-hexylene, 3-oxa-hexylene, 1,3-dioxa-hexylene, 1,4-dioxa-hexylene, 1,5-dioxa-hexylene, 1-oxa-heptylene, 2-oxa-heptylene, 1,3-dioxa-heptylene, 1,4-dioxa-heptylene, 1,5-dioxa-heptylene, 1,6-dioxa-heptylene, 1,3,5-trioxa-heptylene, 1-oxa-octylene, 2-oxa-octylene, 3-oxa-octylene, 4-oxa-octylene, 1,3-dioxaoctylene, 1,4-dioxa-nonylene, 1,4-dioxa-decylene, 1,4-dioxa-undecylene and 1,3,5-trioxa-dodecylene.

X is -O-, -S-, -COO-, -OCO- or a single bond and in particular -O-, -COO-, -OCO- or a single bond. In case X is -O-, -S- or -COO-, the adjacent CH₂-group of Q is not replaced by -O-.

Z¹ is independently from each other -CH₂CH₂- or a single bond.

R² can be an alkyl radical with up to 15 C atoms which is unsubstituted, mono- or polysubstituted by halogen, it also being possible for one or more CH₂ groups in these radicals to be replaced, in each case independently from one another, by -O-, -S-, -CO-, -OCO-, -COO- or -O-COO- in such a manner that oxygen atoms are not linked directly to one another.

If R² is an alkyl radical or alkoxy radical, it may be straight-chain or branched. Preferably, it is straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy or octoxy, and furthermore methyl, nonyl, decyl, undecyl, dodecyl, tridecyl, tetradecyl, pentadecyl, methoxy, nonoxy, decoxy, undecoxy, dodecoxy, tridecoxy or tetradecoxy.

If R² is oxaalkyl, it is preferably straight-chain 2-oxapropyl (= methoxymethyl), 2-oxabutyl (= ethoxymethyl) or 3-oxabutyl (= 2-methoxyethyl), 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, 2-, 3-, 4-, 5-, 6-, 7-, 8- or 9-oxadecyl.

Preferred branched radicals R² are isopropyl, 2-butyl (= 1-methylpropyl), isobutyl (= 2-methylpropyl), 2-methylbutyl, isopentyl, (= 3-methylbutyl), 2-methylpentyl, 3-methylpentyl, 2-ethylhexyl, 2-propylpentyl, 2-octyl, isopropoxy, 2-methylpropoxy, 2-methylbutoxy, 3-methylbutoxy, 2-methyl-pentoxy, 3-methyl-pentoxy, 2-ethylhexoxy, 1-methylhexoxy, 2-octyloxy, 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 5-methylheptyloxycarbonyl, 2-methylbutyryloxy, 3-methylvaleryloxy, 4-methylhexanoyloxy, 2-chloropropionyloxy, 2-chloro-3-methylbutyryloxy, 2-chloro-4-methylvaleryloxy, 2-chloro-3-methylvaleryloxy, 2-methyl-3-oxypentyl, 2-methyl-3 -oxahexyl.

R² can also be a polar terminal group and in particular -CN, -Cl or F; R² can also be -(L)-C_{d}HₑF_{2d+1-e} wherein L is a single bond, -O- or -S-, d is 1 or 2 and e is 0, 1, 2, 3, 4 or 5.

R² can also have one of the meanings given for R¹-Q-X- above. In case R² is an - optionally substituted - alkyl radical, R¹ preferable is a vinyl or acrylate group while in case R² is R¹-Q-X, all meanings given above for R¹ are preferred.

Reactive liquid crystalline compounds exhibiting two reactive groups R¹ which can be chosen independently from each other, are preferred; especially preferred are compounds exhibiting two identical reactive groups.

The reactive liquid crystalline compounds according to formula I and according to the preferred subclasses can be prepared by methods which are known per se and which are described, for example, in standard works of organic chemistry such as, for example, Houben-Weyl, Methoden der Organischen Chemie, Thieme-Verlag, Stuttgart. Some specific methods can be taken from the examples.

Methods to introduce the reactive group R¹ as a substituent are well-known in literature. A preferred method to introduce an acrylate group as a terminal substituent into a rod-like molecule is described in examples 1-3.

Terminal vinyl ether groups can be introduced, for example, by transetherification reactions. The alcohols are treated with butylvinyl ether in dichloromethane in the presence of 1,10-phenanthroline palladium (II) diacetate as is described in V. Perec, M. Lee and H. Jonsson, J. Polym. Sci. Part A, 1991, 29, 327-337.

A thiol group can be introduced, for example, by converting an aliphatic alcohol into a thiol; see, for example, Lucien and Nouveau, J. Chem., 1979, 3, 15.

The "ene" group can be introduced by directly alkylating the core with a suitable ω-bromo-α-alkene.

The reaction methods mentioned are briefly summarized in the following synthetic tree:

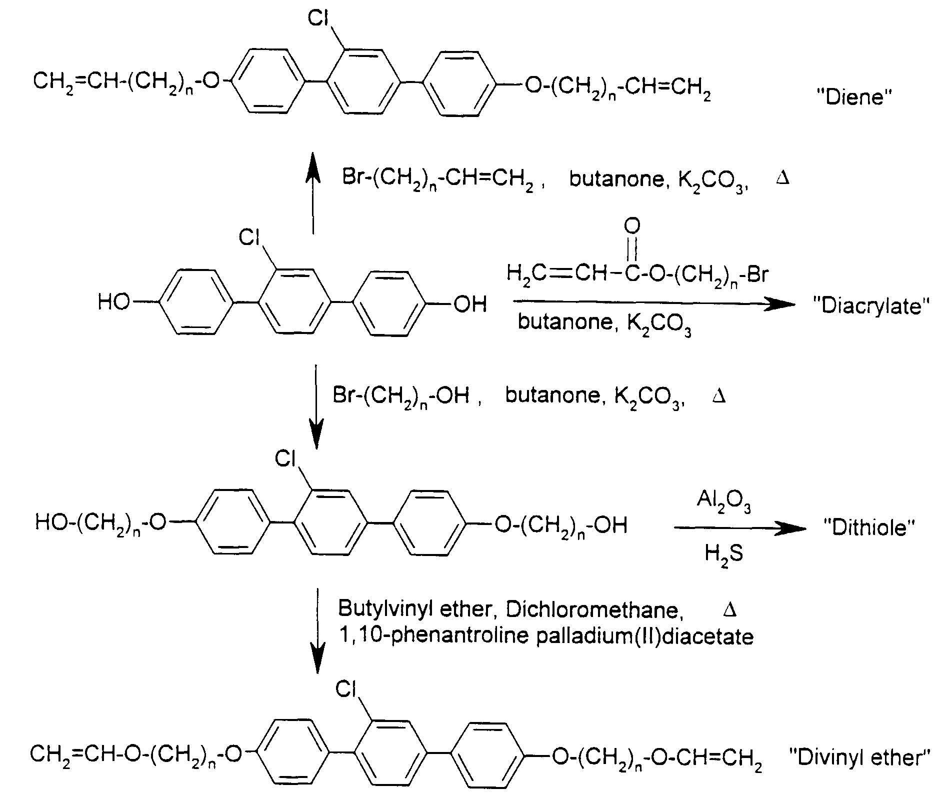

The reaction schemes mentioned above are to illustrate the invention without restricting it. The expert can choose other reaction methods without any inventive efforts.

In the following and in the preceding, all percentages given are percentages by weight. Temperatures are given in degrees Celsius.

The following examples are intended to illustrate the invention without restricting it.

### Example 1

The reactive liquid crystalline compound (1) 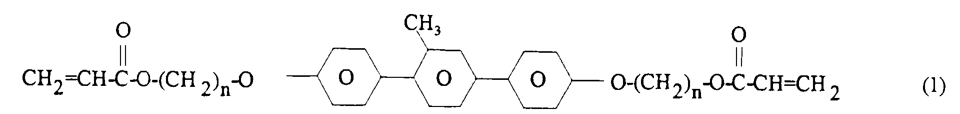 is prepared via the sequence of reaction steps shown in diagram 1.

Compound (1) exhibits the following phase sequence:
n=3: K 73 N 80 I
n = 6: K 69 S_{A} 75 N 78.6 I

### Example 2

The reactive liquid crystalline compound (2) 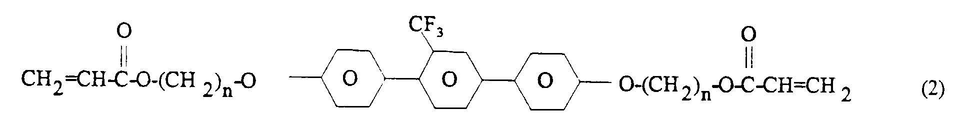 is prepared via the sequence of reaction steps shown in diagram 2.

### Example 5

The reactive liquid crystalline compound (5) 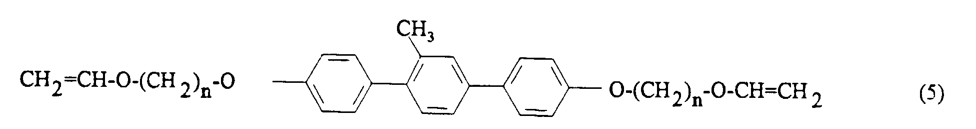 is prepared by reacting the compound obtained in step 3 of diagram 1 according to diagram 4.

Compound (5) exhibits the following phase sequence.
n = 3: K 74.4 (S 37.7 N 52.6) I
n = 6: K 79.4 S 89.6 N 111.4 I

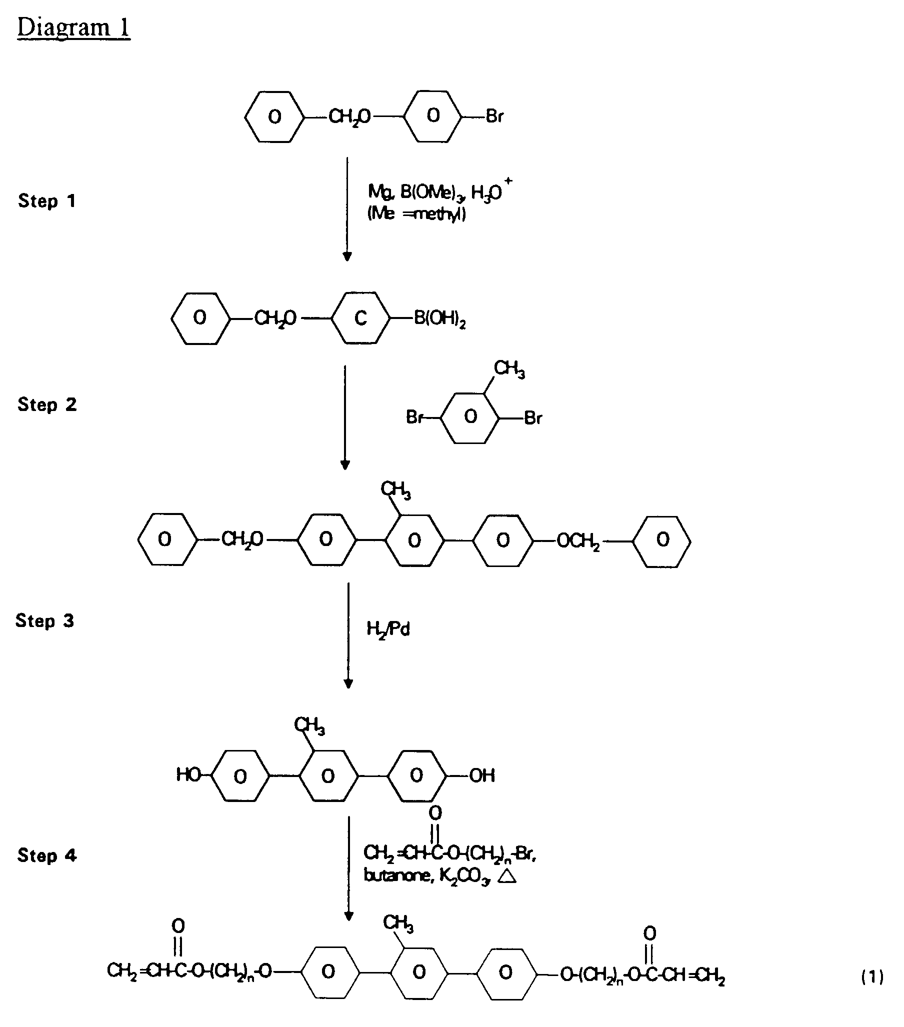

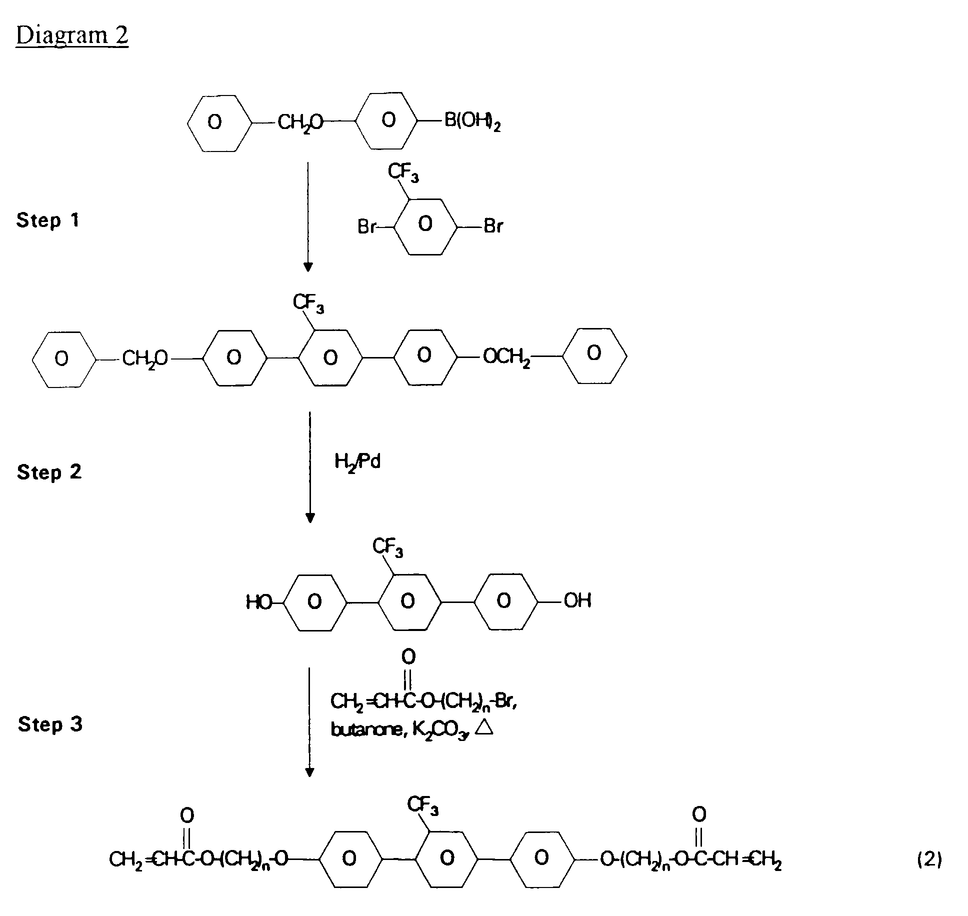

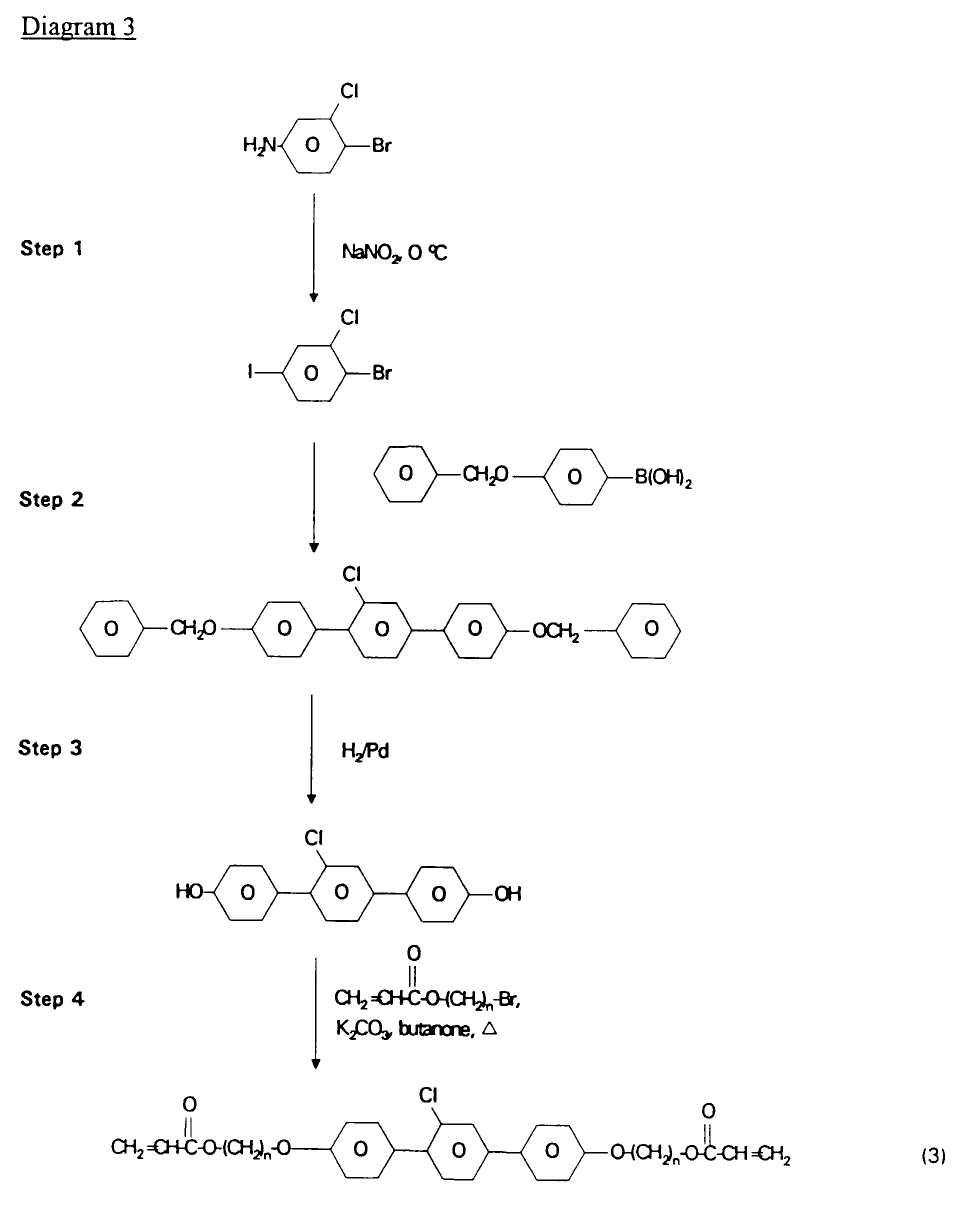

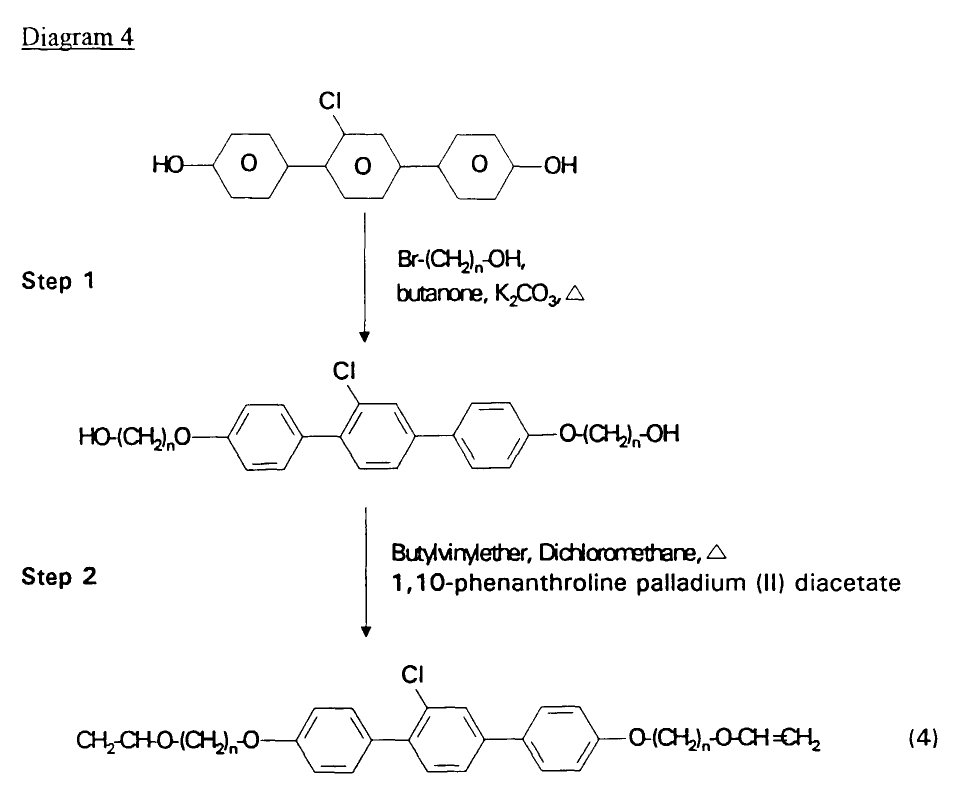

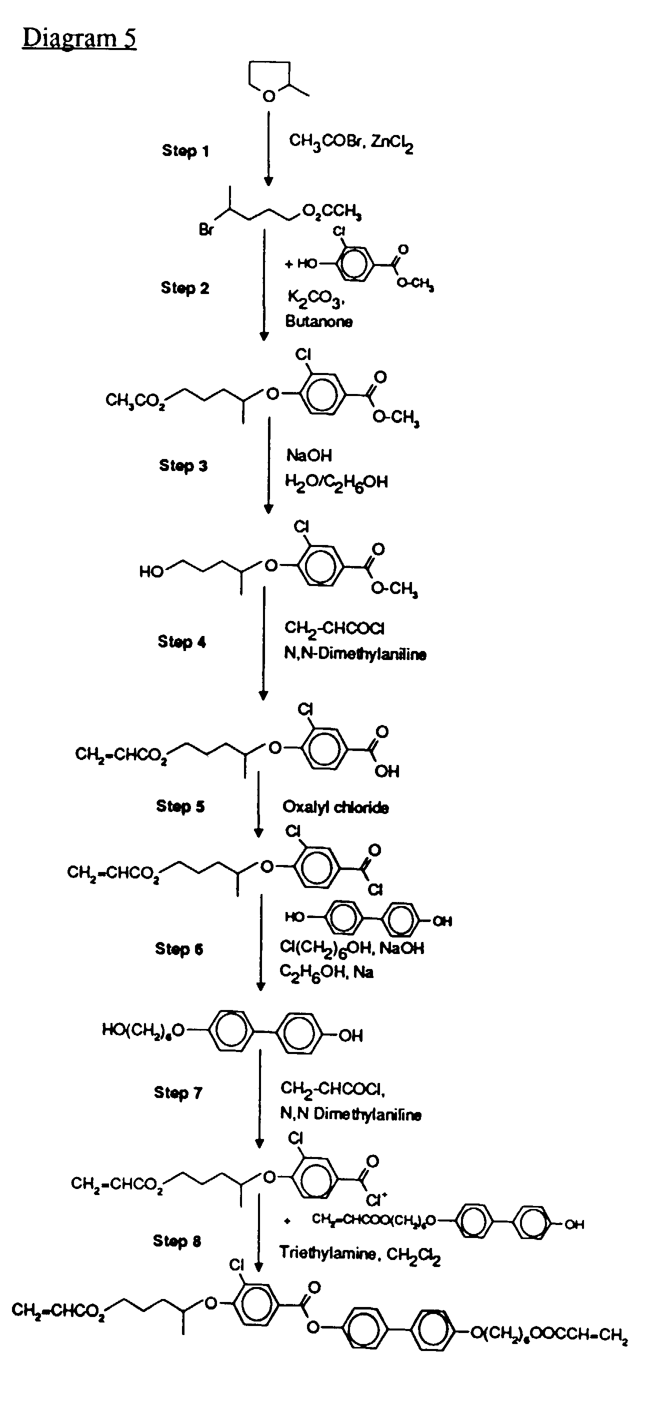

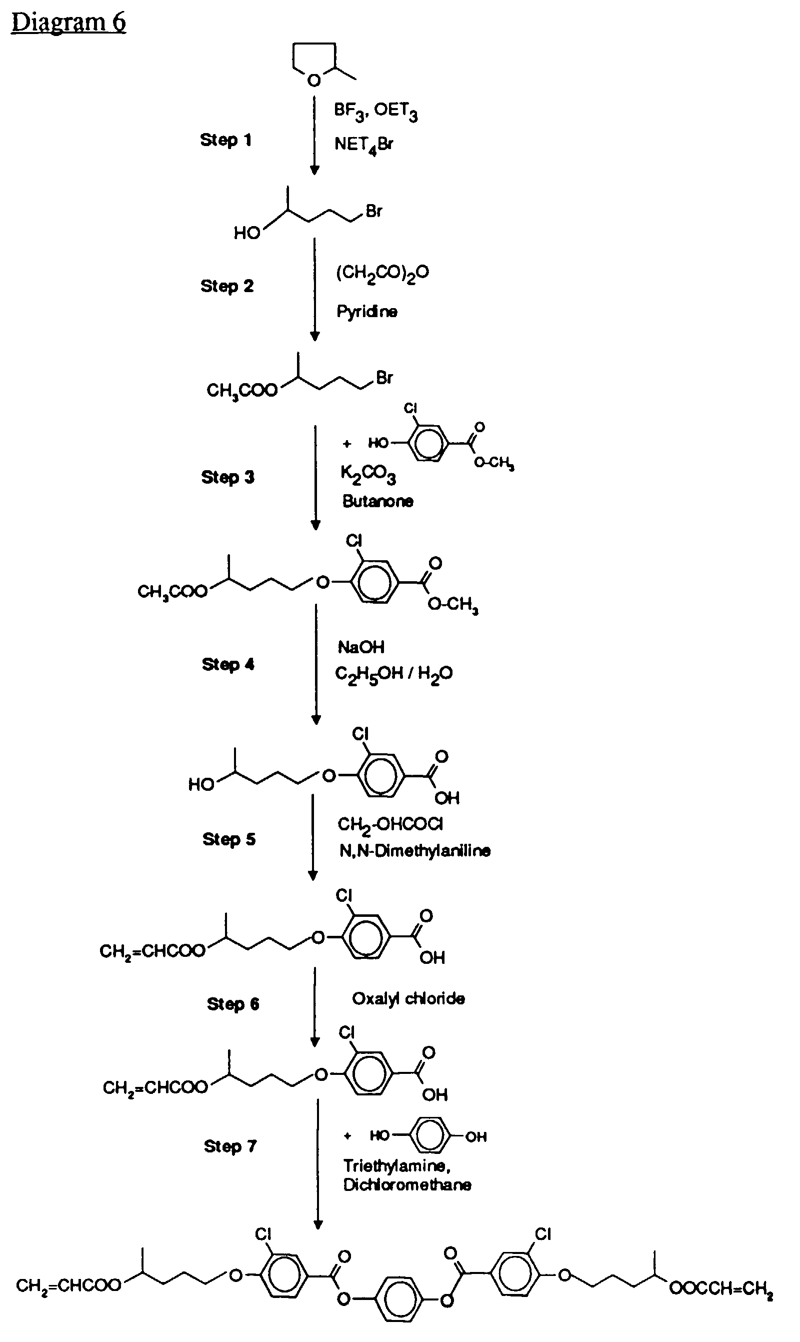

## Claims

1. Reactive liquid crystal compound of formula I
R¹-P-X-(A¹-Z¹)ₙ-A²-R² I
wherein
R¹ is CH₂=CW¹-COO-, CH₂=CH-, 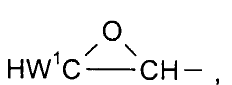 HW¹N- or HS-CH₂-(CH₂)ₘ-COO-, with W¹ being H, Cl or alkyl with 1-5 C-atoms and m being 1-7,
P is alkylene with up to 12 C atoms, it being also possible for one or more CH₂ groups to be replaced by O in such a manner that oxygen atoms are not directly linked to one another,
X is -O-, -S-, -COO-, -OCO- or a single bond,
R² is an alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not linked directly to one another, or alternatively R² has one of the meanings given for R¹-P-X or is CN, F, Cl or -V-Q-T wherein V is -O-, -S- or a single bond, Q is -CH₂-, CHF-, -CF₂-, and T is F or Cl,
n is 1 or 2,
A¹, A² are independently from each other 1,4-phenylene which is unsubstituted or mono- or polysubstituted by F, Cl, CN or -W²-CᵣHₛF₂ᵣ₊₁₋ₛ, and one of A¹ and A² may also denote 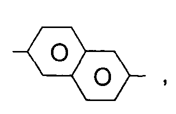 pyridine-2,5-diyl or pyrimidine-2,5-diyl,
Z¹ is independently from each other -CH₂-CH₂- or a single bond, in case n=2 both the moieties A¹ as well as both the moieties Z¹ are having the meaning given above, independently from each other,
r is 1 or 2,
s is 0, 1, 2, 3, 4 or 5,
W² is a single bond, -O-, -S- or -CO-,
with the provisos
(i) that at least one of A¹ and A² is 1,4-phenylene which is mono- or polysubstituted by Cl, CN or -W²-CᵣHₛF₂ᵣ₊₁₋ₛ, it also being possible for this substituted 1,4-phenylene group to be additionally substituted by one or more F-atoms, and
(ii) that in case n=2 at least one of A¹ and A² is double substituted or at least two of A¹ and A² are substituted,
characterized in that said compound is laterally di- or higher substituted by CN or -W²-CᵣHₛF₂ᵣ₊₁₋ₛ.

2. Reactive liquid crystal compound according to claim 1, characterized in that -A¹-Z¹-A¹-Z¹-A²- is 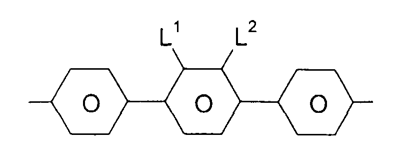 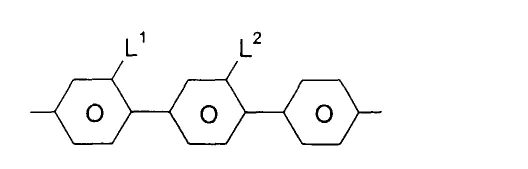 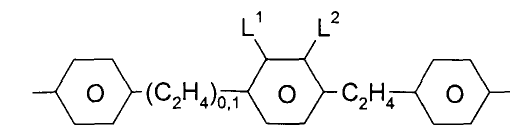 wherein L¹ and L² are independently from each other -CN or-W²-CᵣHₛF₂ᵣ₊₁₋ₛ, and W², r and s have the meanings of claim 1.

3. Reactive liquid crystal compound according to claim 1 or 2, characterized in that it is laterally di- or higher substituted by -CN, -CH₃, -OCH₃, -CF₃ or -OCF₃.

4. Reactive liquid crystal compound of formula I2
R¹-P-X-A¹-Z¹-A²-R² I2
wherein
R¹ is CH₂=CW¹-COO-, CH₂=CH-, 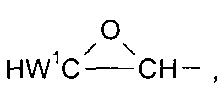 HW¹N- or HS-CH₂-(CH₂)ₘ-COO-, with W being H, Cl or alkyl with 1-5 C-atoms and m being 1-7,
P is alkylene with up to 12 C atoms, it being also possible for one or more CH₂ groups to be replaced by O in such a manner that oxygen atoms are not directly linked to one another,
X is -O-, -S-, -COO-, -OCO- or a single bond,
R² is an alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not linked directly to one another, or alternatively R² has one of the meanings given for R¹-P-X or is CN, F, Cl or -V-Q-T wherein V is -O-, -S- or a single bond, Q is -CH₂-, -CHF-, -CF₂-, and T is F or Cl,
A¹, A² are independently from each other 1,4-phenylene which is unsubstituted or mono- or polysubstituted by F, Cl, CN or -W²-CᵣHₛF₂ᵣ₊₁₋ₛ, and one of A¹ and A² may also denote pyridine-2,5-diyl or pyrimidine-2,5-diyl,
Z¹ is independently from each other -CH₂-CH₂- or a single bond,
r is 1 or 2,
s is 0, 1, 2, 3, 4 or 5,
W² is a single bond, -O-, -S- or -CO-,
with the proviso that at least one of A¹ and A² is 1,4-phenylene which is monosubstituted by Cl, CN or -W²-CᵣHₛF₂ᵣ₊₁₋ₛ, it also being possible for this substituted 1,4-phenylene group to be additionally substituted by one or more F-atoms,
characterized in that said compound is laterally monosubstituted by -Cl, -CN or -W²-CᵣHₛF₂ᵣ₊₁₋ₛ.

5. Reactive liquid crystal compound of formula I3
R¹-P-X-A¹-Z¹-A¹-Z¹-A²-R² I3
wherein
R¹ is CH₂=CW¹-COO-, CH₂=CH-, 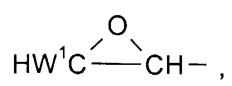 HW¹N- or HS-CH₂-(CH₂)ₘ-COO-, with W¹ being H, Cl or alkyl with 1-5 C-atoms and m being 1-7,
P is alkylene with up to 12 C atoms, it being also possible for one or more CH₂ groups to be replaced by O in such a manner that oxygen atoms are not directly linked to one another,
X is -O-, -S-, -COO-, -OCO- or a single bond,
R² is an alkyl radical with up to 15 C atoms which is unsubstituted, mono- or polysubstituted by halogen, it being also possible for one or more CH₂ groups in these radicals to be replaced, in each case independently of one another, by -O-, -S-, -CO-, -O-CO-, -CO-O- or -O-CO-O- in such a manner that oxygen atoms are not linked directly to one another, or alternatively R² has one of the meanings given for R¹-P-X or is CN, F, Cl or -V-Q-T wherein V is -O-, -S- or a single bond, Q is -CH₂-, -CHF-, -CF₂-, and T is F or Cl,
A¹, A² are independently from each other 1,4-phenylene which is unsubstituted or mono- or polysubstituted by F, Cl, CN or -W²-CᵣHₛF₂ᵣ₊₁₋ₛ, and one of A¹ and A² may also denote 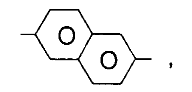 pyridine-2,5-diyl or pyrimidine-2,5-diyl,
Z¹ is independently from each other -CH₂-CH₂- or a single bond, in case n=2 both the moieties A¹ as well as both the moieties Z¹ are having the meaning given above, independently from each other,
r is 1 or 2,
s is 0, 1, 2, 3, 4 or 5,
W² is a single bond, -O-, -S- or -CO-,
with the proviso that at least one of A¹ and A² is 1,4-phenylene which is mono- or polysubstituted by Cl, CN or -W²-CᵣHₛF₂ᵣ₊₁₋ₛ, it also being possible for this substituted 1,4-phenylene group to be additionally substituted by one or more F-atoms,
characterized in that said compound is laterally monosubstituted by Cl, -CN or -W²-CᵣHₛF₂ᵣ₊₁₋ₛ.
with the proviso that compounds being only monosubstituted by Cl are excluded.

6. Reactive liquid crystal compound according to claim 5,
characterized in that -A¹-Z¹-A¹-Z¹-A²- is 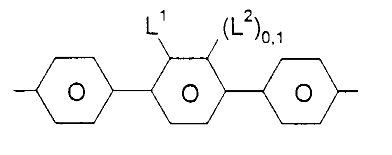 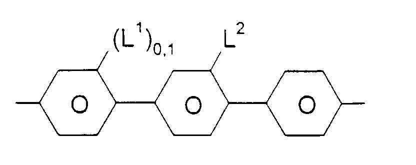 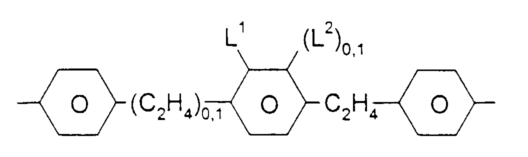 wherein L¹ and L² are independently from each other -F, -Cl,-CN or -W²-CᵣHₛF₂ᵣ₊₁₋ₛ, and W², r and s have the meanings of claim 1.

7. Reactive liquid crystal compound according to claim 4, 5 or 6, characterized in that it is laterally monosubstituted by -CH₃, -O-CH₃, -CF₃, -OCF₃, -CH₂F, OCH₂F, -OHF₂, -OCHF₂, -C₂H₅, -S-CH₃ or -S-CF₃.

8. Reactive liquid crystal compound according to any of claims 1 to 7, characterized in that R² has one of the meanings of R¹-P-X-in formula I.

9. Reactive liquid crystal compound according to any of claims 1 to 8, characterized in that R¹ is vinyl or acrylate.

10. Use of liquid crystal materials according to any of claims 1 to 8 in electrooptical scattering systems or for the preparation of oriented liquid crystal polymers.

## Patentansprüche

1. Reaktionsfähige Flüssigkristallverbindung der Formel I
R¹-P-X- (A¹-Z¹)ₙ-A²-R² I
worin
R¹ CH₂=CW¹-COO-, CH₂=CH-, 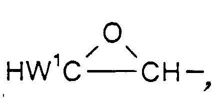 HW¹N- oder HS-CH₂-(CH₂)ₘ-COO- mit W¹ gleich H, Cl oder Alkyl mit 1-5 C-Atomen und m gleich 1-7,
P Alkylen mit bis zu 12 C-Atomen, wobei eine oder mehrere CH₂-Gruppen auch durch O so ersetzt sein können, daß Sauerstoffatome nicht direkt miteinander verknüpft sind,
X -O-, -S-, -COO-, -OCO- oder eine Einfachbindung,
R² einen Alkylrest mit bis zu 15 C-Atomen bedeuten, der gegebenenfalls durch Halogen einfach oder mehrfach substituiert ist, wobei eine oder mehrere CH₂-Gruppen in diesen Resten jeweils unabhängig voneinander auch durch -O-, -S-, -CO-, -O-CO-, -CO-O- oder -O-CO-O- so ersetzt sein können, daß Sauerstoffatome nicht direkt miteinander verknüpft sind, oder wahlweise besitzt R² eine der für R¹-P-X angegebenen Bedeutungen oder bedeutet CN, F, Cl oder -V-Q-T, worin V -O-, -S- oder eine Einfachbindung, Q -CH₂-, -CHF-, -CF₂- und T F oder Cl bedeuten,
n 1 oder 2,
A¹ und A² jeweils unabhängig voneinander 1,4-Phenylen, das gegebenenfalls durch F, Cl, CN oder -W²-CᵣHₛF₂ᵣ₊₁₋ₛ einfach oder mehrfach substituiert ist, und einer der Reste A¹ und A² auch 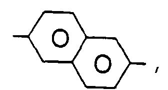 Pyridin-2,5-diyl oder Pyrimidin-2,5-diyl bedeuten kann,
die Reste Z¹ unabhängig voneinander -CH₂-CH₂- oder eine Einfachbindung, wobei, falls n=2 ist, beide Gruppierungen A¹ sowie beide Gruppierungen Z¹ jeweils unabhängig voneinander die obenangegebene Bedeutung besitzen,
r 1 oder 2,
s 0, 1, 2, 3, 4 oder 5 und
W² eine Einfachbindung, -O-, -S- oder -CO- bedeuten,
mit den Maßgaben,
(i) daß mindestens einer der Reste A¹ und A² 1,4-Phenylen bedeutet, das durch Cl, CN oder -W²-CᵣHₛF₂ᵣ₊₁₋ₛ einfach oder mehrfach substituiert ist, wobei diese substituierte 1,4-Phenylengruppe zusätzlich durch ein oder mehrere F-Atome substituiert sein kann, und
(ii) daß, falls n=2 ist, mindestens einer der Reste A¹ und A² zweifach substituiert ist oder mindestens zwei der Reste A¹ und A² substituiert sind,
dadurch gekennzeichnet, daß die genannte Verbindung durch CN oder -W²-CᵣHₛF₂ᵣ₊₁₋ₛ zweifach oder mehr als zweifach seitensubstituiert ist.

2. Reaktionsfähige Flüssigkristallverbindung nach Anspruch 1, dadurch gekennzeichnet, daß A¹-Z¹-A¹-Z¹-A²- 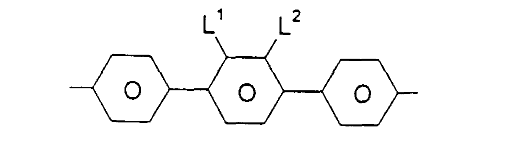 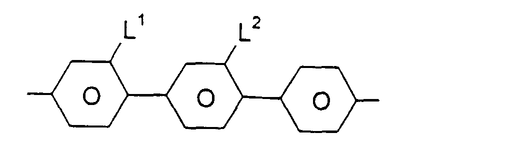 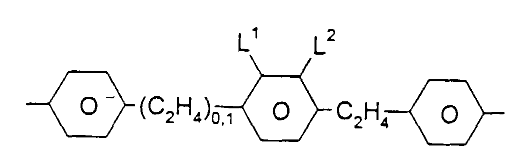 bedeutet,
worin L¹ und L² unabhängig voneinander -CN oder -W²-CᵣHₛF₂ᵣ₊₁₋ₛ bedeuten und W², r und s die Bedeutungen von Anspruch 1 besitzen.

3. Reaktionsfähige Flüssigkristallverbindung nach Anspruch 1 oder 2, dadurch gekennzeichnet, daß sie durch -CN, -CH₃, -OCH₃, -CF₃ oder -OCF₃ zweifach oder mehr als zweifach seitensubstituiert ist.

4. Reaktionsfähige Flüssigkristallverbindung der Formel I2
R¹-P-X-A¹-Z¹-A²-R² I2
worin
R¹ CH₂=CW¹-COO-, CH₂=CH-, 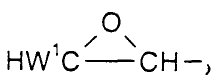 HW¹N- oder HS-CH₂-(CH₂)ₘ-COO- mit W gleich H, Cl oder Alkyl mit 1-5 C-Atomen und m gleich 1-7,
P Alkylen mit bis zu 12 C-Atomen, wobei eine oder mehrere CH₂-Gruppen auch durch O so ersetzt sein können, daß Sauerstoffatome nicht direkt miteinander verknüpft sind,
X -O-, -S-, -COO-, -OCO- oder eine Einfachbindung,
R² einen Alkylrest mit bis zu 15 C-Atomen bedeuten, der gegebenenfalls durch Halogen einfach oder mehrfach substituiert ist, wobei eine oder mehrere CH₂-Gruppen in diesen Resten jeweils unabhängig voneinander auch durch -O-, -S-, -CO-, -O-CO-, -CO-O- oder -O-CO-O- so ersetzt sein können, daß Sauerstoffatome nicht direkt miteinander verknüpft sind, oder wahlweise besitzt R² eine der für R¹-P-X angegebenen Bedeutungen oder bedeutet CN, F, Cl oder -V-Q-T, worin V -O-, -S- oder eine Einfachbindung, Q -CH₂-, -CHF-, -CF₂- und T F oder Cl bedeuten,
A¹ und A² jeweils unabhängig voneinander 1,4-Phenylen bedeuten, das gegebenenfalls durch F, Cl, CN oder -W²-CᵣHₛF₂ᵣ₊₁₋ₛ einfach oder mehrfach substituiert ist, und einer der Reste A¹ und A² auch Pyridin-2,5-diyl oder Pyrimidin-2,5-diyl bedeuten kann,
die Reste Z¹ unabhängig voneinander -CH₂-CH₂- oder eine Einfachbindung,
r 1 oder 2,
s 0, 1, 2, 3, 4 oder 5 und
W² eine Einfachbindung, -O-, -S- oder -CO- bedeuten,
mit der Maßgabe, daß mindestens einer der Reste A¹ und A² 1,4-Phenylen bedeutet, das durch Cl, CN oder -W²-CᵣHₛF₂ᵣ₊₁₋ₛ einfach substituiert ist, wobei diese substituierte 1,4-Phenylengruppe zusätzlich durch ein oder mehrere F-Atome substituiert sein kann,
dadurch gekennzeichnet, daß die genannte Verbindung durch -Cl, -CN oder -W²-CᵣHₛF₂ᵣ₊₁₋ₛ einfach seitensubstituiert ist.

5. Reaktionsfähige Flüssigkristallverbindung der Formel I3
R¹-P-X-A¹-Z¹-A¹-Z¹-A²-R² I3
worin
R¹ CH₂=CW¹-COO-, CH₂=CH-, 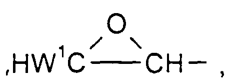 HW¹N- oder HS-CH₂-(CH₂)ₘ-COO- mit W¹ gleich H, Cl oder Alkyl mit 1-5 C-Atomen und m gleich 1-7,
P Alkylen mit bis zu 12 C-Atomen, wobei eine oder mehrere CH₂-Gruppen auch durch O so ersetzt sein können, daß Sauerstoffatome nicht direkt miteinander verknüpft sind,
X -O-, -S-, -COO-, -OCO- oder eine Einfachbindung,
R² einen Alkylrest mit bis zu 15 C-Atomen bedeuten, der gegebenenfalls durch Halogen einfach oder mehrfach substituiert ist, wobei eine oder mehrere CH2-Gruppen in diesen Resten jeweils unabhängig voneinander auch durch -O-, -S-, -CO-, -O-CO-, -CO-O- oder -O-CO-O- so ersetzt sein können, daß Sauerstoffatome nicht direkt miteinander verknüpft sind, oder wahlweise besitzt R² eine der für R¹-P-X angegebenen Bedeutungen oder bedeutet CN, F, Cl oder -V-Q-T, worin V -O-, -S- oder eine Einfachbindung, Q -CH₂-, -CHF-, -CF₂- und T F oder Cl bedeuten,
A¹ und A² jeweils unabhängig voneinander 1,4-Phenylen, das gegebenenfalls durch F, Cl, CN oder -W²-CᵣHₛF₂ᵣ₊₁₋ₛ einfach oder mehrfach substituiert ist, und einer der Reste A¹ und A² auch 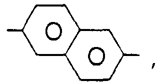 Pyridin-2,5-diyl oder Pyrimidin-2,5-yl bedeuten kann,
die Reste Z¹ unabhängig voneinander -CH₂-CH₂- oder eine Einfachbindung, wobei, falls n=2 ist, beide Gruppierungen A¹ sowie beide Gruppierungen Z¹ jeweils unabhängig voneinander die obenangegebene Bedeutung besitzen,
r 1 oder 2,
s 0, 1, 2, 3, 4 oder 5 und
W² eine Einfachbindung, -O-, -S- oder -CO- bedeuten,
mit der Maßgabe, daß mindestens einer der Reste A¹ und A² 1,4-Phenylen bedeutet, das durch Cl, CN oder -W²-CᵣHₛF₂ᵣ₊₁₋ₛ einfach oder mehrfach substituiert ist, wobei diese substituierte 1,4-Phenylengruppe zusätzlich durch ein oder mehrere F-Atome substituiert sein kann,
dadurch gekennzeichnet, daß die genannte Verbindung durch -Cl, -CN oder -W²-CᵣHₛF₂ᵣ₊₁₋ₛ einfach seitensubstituiert ist,
mit der Maßgabe, daß durch Cl nur einfach substituierte Verbindungen ausgenommen sind.

6. Reaktionsfähige Flüssigkristallverbindung nach Anspruch 5, dadurch gekennzeichnet, daß -A¹-Z¹-A¹-Z¹-A²- 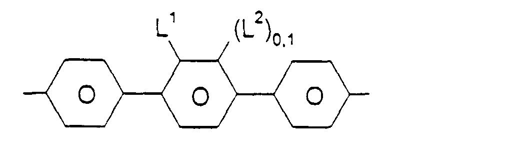 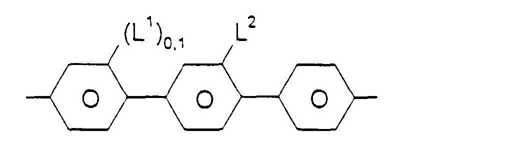 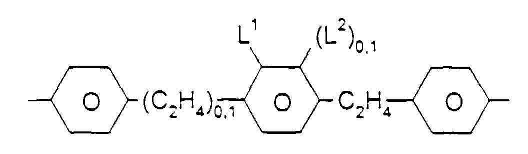 bedeutet,
worin L¹ und L² unabhängig voneinander -F, -Cl, -CN oder -W²-CᵣHₛF₂ᵣ₊₁₋ₛ bedeuten und W², r und s die Bedeutungen von Anspruch 1 besitzen.

7. Reaktionsfähige Flüssigkristallverbindung nach Anspruch 4, 5 oder 6, dadurch gekennzeichnet, daß sie durch -CH₃, -O-CH₃, -CF₃, -OCF₃, -CH₂F, -OCH₂F, -CHF₂, -OCHF₂, -C₂H₅, -S-CH₃ oder -S-CF₃ einfach seitensubstituiert ist.

8. Reaktionsfähige Flüssigkristallverbindung nach einem der Ansprüche 1 bis 7, dadurch gekennzeichnet, daß R² eine der Bedeutungen von R¹-P-X in Formel I besitzt.

9. Reaktionsfähige Flüssigkristallverbindung nach einem der Ansprüche 1 bis 8, dadurch gekennzeichnet, daß R¹ Vinyl oder Acrylat bedeutet.

10. Verwendung von Flüssigkristallmaterialien nach einem der Ansprüche 1 bis 8 in elektrooptischen Streuungssystemen oder zur Herstellung von orientierten Flüssigkristallpolymeren.

## Revendications

1. Composé à cristaux liquides réactif de formule I
R¹-P-X-(A¹-Z¹)ₙ-A²-R² I
dans laquelle
R¹ est CH₂=CW¹-COO-, CH₂=CH-, 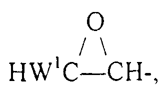 HW¹N- ou HS-CH₂(CH₂)ₘ-COO-, W¹ étant H, Cl ou un groupe alkyle comportant 1-5 atomes de C et m étant 1-7,
P est un groupe alkylène comportant jusqu'à 12 atomes de C, avec la possibilité, pour un ou plusieurs groupes CH₂, d'être remplacés par O de telle manière que les atomes d'oxygène ne soient pas reliés directement les uns aux autres,
X est -O-, -S-, -COO-, -OCO- ou une simple liaison,
R² est un radical alkyle comportant jusqu'à 15 atomes de C, qui est non substitué, mono ou poly-substitué par un halogène, avec la possibilité, pour un ou plusieurs groupes CH₂ dans ces radicaux, d'être remplacés, dans chaque cas indépendamment les uns des autres, par -O-, -S-, -CO-, -O-CO-, -CO-O- ou -O-CO-O-, de telle manière que les atomes d'oxygène ne soient pas reliés directement les uns aux autres, ou bien R² a l'une des significations données pour R¹-P-X- ou est CN, F, Cl ou -V-Q-T, où V est -O-, -S- ou une simple liaison, Q est -CH₂-, -CHF-, -CF₂- et T est F ou Cl,
n est égal à 1 ou 2,
A¹, A² sont, indépendamment l'un de l'autre, un groupement 1,4-phénylène qui est non substitué, mono ou poly-substitué par F, Cl, CN ou -W²-CᵣHₛF₂ᵣ₊₁₋ₛ, et un des radicaux A¹ et A² peut aussi désigner un groupe 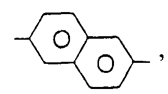 pyridine-2,5-diyle ou pyrimidine-2,5-diyle,
Z¹ est indépendamment des autres un groupe -CH₂-CH₂- ou une simple liaison, dans le cas où n=2, les deux groupements A¹ ainsi que les deux groupements Z¹ ont la signification indiquée ci-dessus, indépendamment l'un de l'autre,
r est égal à 1 ou 2,
s est égal à 0, 1, 2, 3, 4 ou 5,
W² est une simple liaison, -O-, -S- ou -CO-,
à condition
(i) qu'au moins un des radicaux A¹ et A² soit un groupement-1,4-phénylène qui est mono ou poly-substitué par Cl, CN ou -W²-CᵣHₛF₂ᵣ₊₁₋ₛ, avec aussi la possibilité, pour ce groupe 1,4-phénylène substitué, d'être également substitué par un ou plusieurs atomes de F, et
(ii) que, dans le cas où n=2, au moins un des radicaux A¹ et A² soit di-substitué ou qu'au moins deux des radicaux A¹ et A² soient substitués, caractérisé en ce que ledit composé est di-substitué ou poly-substitué latéralement par CN ou -W²-CᵣHₛF₂ᵣ₊₁₋ₛ.

2. Composé à cristaux liquides réactif selon la revendication 1, caractérisé en ce que -A¹-Z¹-A¹-Z¹-A²- est 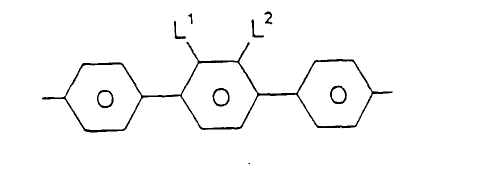 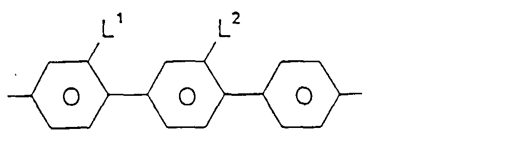 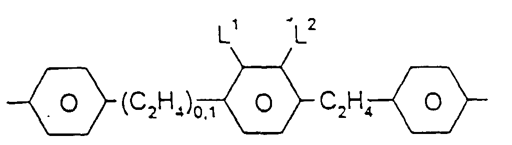 où L¹ et L² sont, indépendamment l'un de l'autre, -CN ou -W²-CᵣHₛF₂ᵣ₊₁₋ₛ, et W², r et s ont les significations données dans la revendication 1.

3. Composé à cristaux liquides réactif selon la revendication 1 ou 2, caractérisé en ce qu'il est di-substitué ou poly-substitué latéralement par -CN-, -CH₃, -OCH₃-, -CF₃ ou -OCF₃.

4. Composé à cristaux liquides réactif de formule I2
R¹-P-X-A¹-Z¹-A²-R² I2
dans laquelle
R¹ est CH₂=CW¹-COO-, CH₂=CH-, 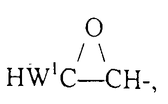 HW¹N- ou HS-CH₂(CH₂)ₘ-COO-, W¹ étant H, Cl ou un groupe alkyle comportant 1-5 atomes de C et m étant 1-7,
P est un groupe alkylène comportant jusqu'à 12 atomes de C, avec la possibilité, pour un ou plusieurs groupes CH₂, d'être remplacés par O de telle manière que les atomes d'oxygène ne soient pas reliés directement les uns aux autres,
X est -O-, -S-, -COO-, -OCO- ou une simple liaison,
R² est un radical alkyle comportant jusqu'à 15 atomes de C, qui est non substitué, mono ou poly-substitué par un halogène, avec la possibilité, pour un ou plusieurs groupes CH₂ dans ces radicaux, d'être remplacés, dans chaque cas indépendamment les uns des autres, par -O-, -S-, -CO-, -O-CO-, -CO-O- ou -O-CO-O-, de telle manière que les atomes d'oxygène ne soient pas reliés directement les uns aux autres, ou bien R² a l'une des significations données pour R'-P-X- ou est CN, F, Cl ou -V-Q-T, où V est -O-, -S- ou une simple liaison, Q est -CH₂-, -CHF-, -CF₂- et T est F ou Cl,
A¹, A² sont, indépendamment l'un de l'autre, un groupement 1,4-phénylène qui est non substitué ou mono ou poly-substitué par F, Cl, CN ou -W²-CᵣHₛF₂ᵣ₊₁₋ₛ, et un des radicaux A¹ et A² peut aussi désigner un groupe pyridine-2,5-diyle ou pyrimidine-2,5-diyle,
Z¹ est indépendamment des autres un groupe -CH₂-CH₂- ou une simple liaison,
r est égal à 1 ou 2,
s est égal à 0, 1, 2, 3, 4 ou 5,
W² est une simple liaison, -O-, -S- ou -CO-,
à condition qu'au moins un des radicaux A¹ et A² soit un groupement 1,4-phénylène qui est mono-substitué par Cl, CN ou -W²-CᵣHₛF₂ᵣ₊₁₋ₛ, avec aussi la possibilité, pour ce groupe 1,4-phénylène substitué, d'être également substitué par un ou plusieurs atomes de F,
caractérisé en ce que ledit composé est mono-substitué latéralement par -Cl, -CN ou -W²-CᵣHₛF₂ᵣ₊₁₋ₛ.

5. Composé à cristaux liquides réactif de formule I3
R¹-P-X-A¹-Z¹-A¹-Z¹-A²-R² I3
dans laquelle
R¹ est CH₂=CW¹-COO-, CH₂=CH-, 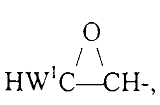 HW¹N- ou
HS-CH₂(CH₂)ₘ-COO-, W¹ étant H, Cl ou un groupe alkyle comportant 1-5 atomes de C et m étant 1-7,
P est un groupe alkylène comportant jusqu'à 12 atomes de C, avec la possibilité, pour un ou plusieurs groupes CH₂, d'être remplacés par O de telle manière que les atomes d'oxygène ne soient pas reliés directement les uns aux autres,
X est -O-, -S-, -COO-, -OCO- ou une simple liaison,
R² est un radical alkyle comportant jusqu'à 15 atomes de C, qui est non substitué, mono ou poly-substitué par un halogène, avec la possibilité, pour un ou plusieurs groupes CH₂ dans ces radicaux, d'être remplacés, dans chaque cas indépendamment les uns des autres, par -O-, -S-, -CO-, -O-CO-, -CO-O- ou -O-CO-O-, de telle manière que les atomes d'oxygène ne soient pas reliés directement les uns aux autres, ou bien R² a l'une des significations données pour R¹-P-X- ou est CN, F, Cl ou -V-Q-T, où V est -O-, -S- ou une simple liaison, Q est -CH₂-, -CHF-, -CF₂- et T est F ou Cl,
A¹, A² sont, indépendamment l'un de l'autre, un groupement 1,4-phénylène qui est non substitué ou mono ou poly-substitué par F, Cl, CN ou -W²-CᵣHₛF₂ᵣ₊₁₋ₛ, et un des radicaux A¹ et A² peut aussi désigner un groupe 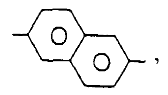 pyridine-2,5-diyle ou pyrimidine-2,5-diyle,
Z¹ est indépendamment des autres un groupe -CH₂-CH₂- ou une simple liaison, dans le cas où n=2, les deux groupements A¹ ainsi que les deux groupements Z¹ ont la signification indiquée ci-dessus, indépendamment l'un de l'autre,
r est égal à 1 ou 2,
s est égal à 0, 1, 2, 3, 4 ou 5,
W² est une simple liaison, -O-, -S- ou -CO-,
à condition qu'au moins un des radicaux A¹ et A² soit un groupement 1,4-phénylène qui est mono ou poly-substitué par Cl, CN ou -W²-CᵣHₛF₂ᵣ₊₁₋ₛ, avec aussi la possibilité, pour ce groupe 1,4-phénylène substitué, d'être également substitué par un ou plusieurs atomes de F,
caractérisé en ce que ledit composé est mono-substitué latéralement par Cl, -CN ou -W²-CᵣHₛF₂ᵣ₊₁₋ₛ,
à condition d'exclure les composés seulement mono-substitués par Cl.

6. Composé à cristaux liquides réactif selon la revendication 5, caractérisé en ce que -A¹-Z¹-A¹-Z¹-A²- est 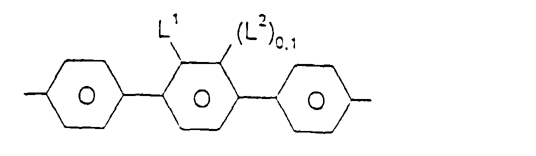 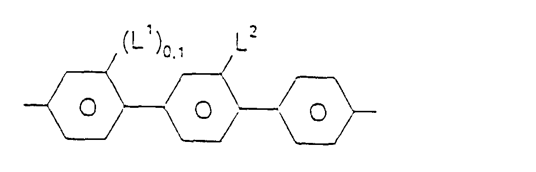 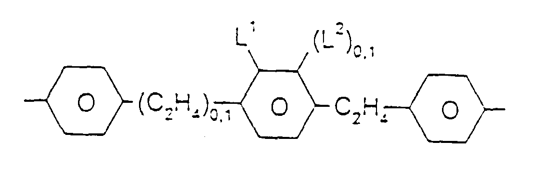 où L¹ et L² sont, indépendamment l'un de l'autre, -F, -Cl, -CN ou -W²-CᵣHₛF₂ᵣ₊₁₋ₛ, et W², r et s ont les significations données dans la revendication 1.

7. Composé à cristaux liquides réactif selon la revendication 4, 5 ou 6, caractérisé en ce qu'il est mono-substitué latéralement par -CH₃, -O-CH₃-, -CF₃, -OCF₃, -CH₂F, OCH₂F, -CHF₂, -OCHF₂, -C₂H₅, -S-CH₃ ou -S-CF₃.

8. Composé à cristaux liquides réactif selon l'une quelconque des revendications 1 à 7, caractérisé en ce que R² a une des significations de R¹-P-X- dans la formule I.

9. Composé à cristaux liquides réactif selon l'une quelconque des revendications 1 à 8, caractérisé en ce que R¹ est un groupe vinyle ou acrylate.

10. Utilisation de matériaux à cristaux liquides selon l'une quelconque des revendications 1 à 8 dans des systèmes de diffusion électro-optiques ou pour la préparation de polymères à cristaux liquides orientés.
